# EUROPEAN PATENT APPLICATION

(11) **EP 4 464 340 A1**
(43) Date of publication of application: **20.11.2024**
(21) Application number: 22919574.8
(22) Date of filing: 22.01.2022
(51) Int. Cl.: A61K 49/06, A61B 5/055

(54) **CONTRAST AGENT, PREPARATION METHOD FOR CONTRAST AGENT, AND APPLICATION OF CONTRAST AGENT**

(30) Priority: 14.01.2022 CN 202210043497
(71) Applicant: Wu, Shiman, Shanghai 200433 (CN)
(72) Inventor: Wu, Shiman, Shanghai 200433 (CN)
(74) Representative: Ipside
(86) International application number: PCT/CN2022/073329
(87) International publication number: WO 2023/133928

(57) **Abstract**

A contrast agent, a preparation method for the contrast agent, and an application of the contrast agent, aiming to find a more effective implementation solution of a magnetic resonance contrast agent. The preparation method for the contrast agent comprises the following steps: obtaining ex-vivo blood containing oxyhemoglobin, wherein the ex-vivo blood is autologous blood of a subject or qualified ex-vivo blood that has the same blood type of the subject and satisfies clinical standard allogeneic blood transfusion conditions; introducing carbon monoxide into the ex-vivo blood until the oxyhemoglobin is saturated with the carbon monoxide, so as to obtain a contrast agent comprising carboxyhemoglobin. By means of the approach of generating the contrast agent by using the autologous blood or the qualified ex-vivo blood that has the same blood type of the subject and satisfies the clinical standard allogeneic blood transfusion conditions, adverse reactions such as allergies to metal-ion contrast agents are avoided, economic costs are also reduced to a certain extent, and large-scale and diversified popularization and application are facilitated.

## Description

### Technical Field

The present invention relates to the field of a medical contrast agent and in particular to a contrast agent, a preparation method for the contrast agent, and an application of the contrast agent.

### Background of the Invention

In Magnetic Resonance Imaging (MRI), radio frequency pulses are employed to excite the atomic nuclei with non-zero spin in a magnetic field; after the radio frequency pulses are stopped, the nuclei are relaxed; in the relaxation process, signals are collected by using an induction coil, and mathematical images are reconstructed according to a certain mathematical method. Specifically, relaxation refers to that in the MRI process, after the hydrogen nuclei of water molecules in the test body are excited by radio frequency and absorb energy, the macroscopic magnetization vector deflects; when the radio frequency pulses are turned off, the hydrogen nuclei will be rearranged along the direction of outer magnetic field, releasing the energy and returning to the equilibrium state. Clinically used MRIs are mainly based on T₁ relaxation, T₂ relaxation and T₂* relaxation.

In order to enhance the difference of the intensity of image signals, usually it is needed to inject an exogenous contrast agent in the existing MRI process; but the commonly used exogenous contrast agents are mainly gadolinium-based contrast agents, which have the risk of causing renal damage and deposition in the body; moreover, these contrast agents are only temporarily confined to the blood vessels, and after a few cardiac cycles, they will diffuse into the extravascular space, which may cause diagnostic bias. In addition, exogenous contrast agents include iron-based and manganese-based contrast agents; iron-based contrast agents, when rapidly injected, are prone to cause complications such as phlebitis and hypotension, and their costs are higher than gadolinium-based contrast agents; manganese-based contrast agents are neurotoxic to some extent.

### Summary of the Invention

In order to find a more effective implementation solution of a magnetic resonance contrast agent, the following are taken into consideration. Most of the hemoglobin in the blood within human body is oxyhemoglobin (Oxy-Hb). The hemoglobin molecule consists of two alpha (α) and two beta (β) subunits, each containing an iron-containing heme group to which oxygen can bind. Oxy-Hb has weak diamagnetism because the bonding of oxygen results in the absence of unpaired electrons in the configuration around heme iron. However, the affinity between carbon monoxide and hemoglobin is 200-300 times higher than that between oxygen and hemoglobin, so that carbon monoxide binds very easily to hemoglobin; it is difficult to separate hemoglobin and carbon monoxide after they binds together, producing carboxyhemoglobin (HbCO). When the oxyhemoglobin in the blood is converted into the carboxyhemoglobin, When the oxyhemoglobin in the blood is converted into the carboxyhemoglobin, the oxygen in the hemoglobin is separated from the iron ions, and the conformation of the hemoglobin changes, which prevents the surrounding water molecules from approaching the iron ions. Therefore, the resultant carboxyhemoglobin will have four unpaired electrons and thus be paramagnetic, leading to shorter T1, T2 and T2* relaxation times, so that it can serve as a contrast agent for MRI. Therefore, after a portion of blood is drawn and completely saturated with carbon monoxide to produce the carboxyhemoglobin, it can be applied as a contrast agent in enhanced MRI for a subject who provides autologous blood or has the same blood type, avoiding adverse reactions such as allergy to exogenous contrast agents. At the same time, because the carboxyhemoglobin is a macromolecular substance and cannot permeate into the tissue through the vascular endothelial gap, the high or low perfusion signals observed on the magnetic resonance imaging can suggest the vascular nature of a lesion, i.e. hypervascular or hypovascular. It avoids the difficulty in diagnosing a lesion's vascularity due to the extravasation of traditional gadolinium agents into the extravascular space. Therefore, the present invention provides a contrast agent, a preparation method for the contrast agent, and an application of the contrast agent.

### Solution 1:

Provided is a preparation method for the contrast agent, the preparation method for the contrast agent comprises the following steps:
obtaining ex-vivo blood containing oxyhemoglobin, wherein the ex-vivo blood is autologous blood of a subject or qualified ex-vivo blood that has the same blood type of the subject and satisfies clinical standard allogeneic blood transfusion conditions;
introducing carbon monoxide into the ex-vivo blood until the oxyhemoglobin is saturated with the carbon monoxide, so as to obtain a contrast agent comprising carboxyhemoglobin.

Preferably, obtaining ex-vivo blood containing oxyhemoglobin comprises the following step:
collecting ex-vivo blood that meets a preset specification by using preconfigured blood collection devices and venous blood collection method.

Preferably, before introducing carbon monoxide into the ex-vivo blood until the oxyhemoglobin is saturated with the carbon monoxide, it comprises the following step:
providing light protection for the ex-vivo blood.

Preferably, introducing carbon monoxide into the ex-vivo blood until the oxyhemoglobin is saturated with the carbon monoxide comprises the following steps:
generating carbon monoxide by using a carbon monoxide gas generating device;
introducing the carbon monoxide into the ex-vivo blood and allowing the ex-vivo blood not to spill until all of the oxyhemoglobin contained in the ex-vivo blood is saturated with the carbon monoxide, and then stopping the introduction of carbon monoxide.

Preferably, after stopping the introduction of carbon monoxide, it comprises the following step:
discharging undissolved carbon monoxide.

Preferably, discharging undissolved carbon monoxide comprises the following step:
discharging undissolved carbon monoxide by using an inert gas after stopping the introduction of carbon monoxide.

Preferably, the inert gas is nitrogen or helium.

Preferably, after obtaining a contrast agent comprising carboxyhemoglobin, it comprises the following step:
isolating the contrast agent from outside air, and storing it in a preset environment for later use.

### Solution 2:

Provided is a contrast agent, the contrast agent comprises carboxyhemoglobin.

### Solution 3:

A contrast agent according to Solution 2, which can be applied in magnetic resonance imaging examination.

Compared with the prior art, the contrast agent, the preparation method for the contrast agent and the application of the contrast agent provided by the present disclosure have the following beneficial effects:
In the present disclosure, by means of the approach of generating the contrast agent by using the autologous blood or the qualified ex-vivo blood that has the same blood type of the subject and satisfies the clinical standard allogeneic blood transfusion conditions, adverse reactions such as allergies to metal-based contrast agents are avoided, economic costs are also reduced to a certain extent, and large-scale and diversified popularization and application are facilitated.

Additional aspects and advantages of the present invention will be set forth in the following description; these will be obvious from the following description, or be learned by the practice of the invention.

### Brief Description of the Drawings

The above and/or additional aspects and advantages of the present invention will be obvious and understandable from the following description of the embodiments through the use of the accompanying drawings, in which:
FIG. 1 illustrates a flow chart of a preparation method for the contrast agent according to one embodiment of the present disclosure;
FIG. 2 illustrates the magnetic hysteresis loops of the human blood containing carboxyhemoglobin contrast agent and the human blood without contrast agent treatment in an application of the contrast agent according to one more embodiment of the present disclosure.

### Detailed Description of Embodiments

In order to make those skilled in the art better understand the solutions of the present invention, the technical solutions in the embodiments of the disclosure will be clearly and completely described below with reference to the accompanying drawings in the embodiments of the invention.

Some processes described in the Description, Claims and Drawings of the present disclosure include multiple operations shown in a particular order. But it should be understood that these operations may be performed in a different order from the one shown herein or be performed in parallel. The numbers e.g. 101, 103 are used only for distinguishing different operations from each other, and the numbers themselves do not indicate any sequence between the operations. In addition, these processes may include more or fewer operations, and these operations may be performed in sequence or in parallel.

The technical solutions in the embodiments of the present disclosure will be clearly and completely described below with reference to the accompanying drawings in the embodiments of the invention. Obviously, the described embodiments are only some of the embodiments of the invention, rather than all of the embodiments. Based on the embodiments of the invention, all other embodiments obtained by those skilled in the art without creative work shall fall within the protection scope of the present disclosure.

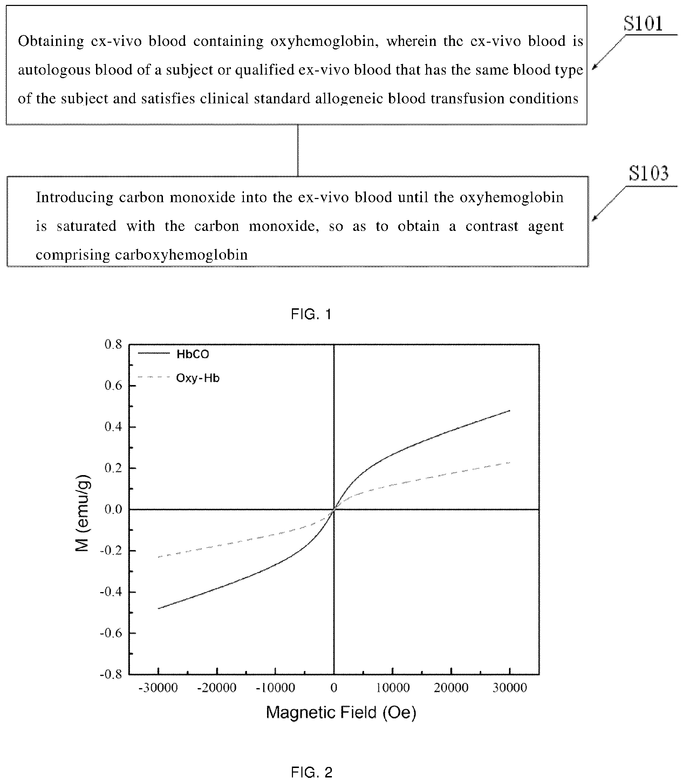

Reference is made to FIG. 1, which illustrates a flow chart of a preparation method for the contrast agent according to one embodiment of the present disclosure. As shown in FIG. 1, the preparation method for the contrast agent according to one embodiment of the invention comprises the following steps:
Step S101: Obtaining ex-vivo blood containing oxyhemoglobin, wherein the ex-vivo blood is autologous blood of a subject or qualified ex-vivo blood that has the same blood type of the subject and satisfies clinical standard allogeneic blood transfusion conditions.

In some embodiments, assuming that the ex-vivo blood is provided by the subject, obtaining ex-vivo blood containing oxyhemoglobin comprises the following step:
collecting ex-vivo blood that meets a preset specification by using preconfigured blood collection devices and venous blood collection method.

In some embodiments, preconfigured blood collection devices include a disposable blood collection needle and a vacuum anticoagulation tube. Specifically, to collect blood, what's needed is just to open the package of a disposable blood collection needle, and then drive the needle to quickly pierce into intravenous cavity in the skin along the vein direction at an acute angle to the skin. When the needle enters the blood vessel, a small amount of blood backflow may be seen. The other end of the blood collection needle is inserted into the vacuum anticoagulation tube. Due to the negative pressure in the vacuum anticoagulation tube, blood automatically flows into the tube. After the preset specification for blood collection is reached, the other end of the blood collection needle is pulled out of the vacuum anticoagulation tube.

In some embodiments, the range of preset specification is generally between 10 ml and 50 ml. However, it should be noted that the preset specification is usually defined based on the blood volume of the subject who will receive an MRI examination. The purpose of doing so is to effectively avoid the occurrence of carboxyhemoglobin poisoning, e.g. 10%-20% of the mild poisoning concentration of carboxyhemoglobin; the instantaneous maximum concentration of carboxyhemoglobin in the blood should be lower than 10% of the minimum mild poisoning concentration.

Exemplarily, assuming that an adult's blood volume is 5,000 ml, the preset specification can be defined as 10 ml. So, in the MRI examination, the instantaneous maximum concentration of carboxyhemoglobin in the blood will be about 2%, far lower than 10%-20% of the mild poisoning concentration of carboxyhemoglobin. Therefore, the carboxyhemoglobin in autologous blood injection is far lower than the poisoning concentration of carboxyhemoglobin. Moreover, after fresh air is inhaled, the carbon monoxide can be dissociated from the carboxyhemoglobin after 8 hours, much shorter than the metabolic cycle of gadolinium contrast agents, i.e. 72 hours. Thus, it ensures the safety of patients. In special cases, if the subject develops a mild adverse reaction related to carboxyhemoglobin and the symptoms do not mitigate after breathing fresh air, hyperbaric oxygen treatment can be performed; if the subject suffers a severe adverse reaction related to carboxyhemoglobin, hyperbaric oxygen treatment can be performed immediately.

Step S103: Introducing carbon monoxide into the ex-vivo blood until the oxyhemoglobin is saturated with the carbon monoxide, so as to obtain a contrast agent comprising carboxyhemoglobin.

In some embodiments, before introducing carbon monoxide into the ex-vivo blood until the oxyhemoglobin is saturated with the carbon monoxide, it comprises the following step:
providing light protection for the ex-vivo blood.

Specifically, to provide light protection for the ex-vivo blood, the vacuum anticoagulation tube can be wrapped with black or opaque adhesive tapes to avoid exposure to light, especially outdoor ultraviolet light. Preferably, special light-protecting vacuum anticoagulation tube can be used.

Preferably, introducing carbon monoxide into the ex-vivo blood until the oxyhemoglobin is saturated with the carbon monoxide comprises the following steps:
generating carbon monoxide by using a carbon monoxide gas generating device;
introducing the carbon monoxide into the ex-vivo blood and allowing the ex-vivo blood not to spill until all of the oxyhemoglobin contained in the ex-vivo blood is saturated with the carbon monoxide, and then stopping the introduction of carbon monoxide.

Exemplarily, to introduce the carbon monoxide into the ex-vivo blood and allow the ex-vivo blood not to spill, the carbon monoxide can be introduced into the ex-vivo blood drawn early and stored in the anticoagulation tube until the oxyhemoglobin contained in the blood is completely saturated with the carbon monoxide.

In some embodiments, the test methods e.g. Raman spectroscopy are employed to determine that the oxyhemoglobin is completely saturated with the carbon monoxide.

In some embodiments, after stopping the introduction of carbon monoxide, it comprises the following step: discharging undissolved carbon monoxide.

In some embodiments, discharging undissolved carbon monoxide comprises the following step: discharging undissolved carbon monoxide by using an inert gas after stopping the introduction of carbon monoxide.

Preferably, the inert gas is nitrogen or helium.

In some embodiments, after obtaining a contrast agent comprising carboxyhemoglobin, it comprises the following step: isolating the contrast agent from outside air, and storing it in a preset environment for later use.

Exemplarily, to isolate the contrast agent from outside air and store it in a preset environment for later use, the stopper of vacuum anticoagulation tube can be tightened to avoid exposure to outside air; it is stored at room temperature i.e. 20°C-30°C for later use within 24 hours; when needed, it can be directly used for contrast-enhanced MRI examination.

Preferably, to isolate the contrast agent from outside air and store it in a preset environment for later use, the stopper of vacuum anticoagulation tube can be tightened to avoid exposure to outside air; it is stored at 4°C within 24 hours; when needed, it can be directly used for contrast-enhanced MRI examination.

Having the same inventive concept as the preparation method for the contrast agent according to one embodiment of the present disclosure, another embodiment of the invention provides a contrast agent, wherein the contrast agent comprises carboxyhemoglobin.

Referring to FIG. 2, having the same inventive concept as the preparation method for the contrast agent according to one embodiment of the present disclosure and the contrast agent provided in another embodiment of the invention, one more embodiment of the invention provides an application of the contrast agent, which can be applied in MRI examination.

Specifically, in the implementation process, it is considered that a conventional enhanced MRI examination is divided into two parts; the first part is to perform conventional sequential scanning for structural imaging for about 5-10 minutes; the second part is to inject the contrast agent and then perform sequential scanning for functional imaging (e.g. perfusion weighted imaging). Therefore, the present disclosure proposes that the preset specification e.g. 10 ml is defined 10 minutes before the patient receives the MRI examination. The blood is stored in an anticoagulation tube, and then the process of preparing methemoglobin is started; the preparation can be done within about 15 minutes. After the patient completes the first part of MRI i.e. conventional scanning, 10 ml of autologous blood treated by carboxyhemoglobin preparation device can be injected to perform the magnetic resonance examination. If the conventional MRI examination and the enhanced MRI are not performed at a time, the carboxyhemoglobin contrast agent can be stored air-tightly; it can be used later for the enhanced MRI on the same day. Alternatively, the qualified ex-vivo blood of a healthy donor that has the same blood type and satisfies clinical standard can be used to prepare carboxyhemoglobin contrast agent for immediate use.

Exemplarily, as shown in FIG. 2, it illustrates the magnetic hysteresis loops of the human blood containing carboxyhemoglobin contrast agent and the human blood without contrast agent treatment in an application of the contrast agent according to one more embodiment of the present disclosure, wherein, HbCO is the human blood containing carboxyhemoglobin contrast agent; Oxy-Hb is the human blood without contrast agent treatment; the magnetic hysteresis loops are the closed magnetization curves that indicate the hysteresis of highly magnetic substances when the magnetic field intensity changes periodically.

As can be seen from FIG. 2, the human blood containing carboxyhemoglobin contrast agent is paramagnetic with magnetic moment of 8.13; while the human blood without contrast agent treatment is not paramagnetic with magnetic moment of 6.46;therefore, one more embodiment of the present disclosure provides an application of the contrast agent which can be used in enhanced MRI.

Compared with the prior art, the contrast agent, the preparation method for the contrast agent and the application of the contrast agent according to the present disclosure have the following beneficial effects:
In the present disclosure, by means of the approach of generating the contrast agent by using the autologous blood or the qualified ex-vivo blood that has the same blood type as the autologous blood and satisfies the clinical allogeneic blood transfusion conditions, adverse reactions such as allergies to metal-based contrast agents are avoided, economic costs are also reduced to a certain extent, and large-scale and diversified popularization and application are facilitated.

The above described are only some of the embodiments of the present disclosure; it should be noted that, for a person of ordinary skill in the art, various improvements and modifications can be made without departing from the principle of the invention; these improvements and modifications shall also be considered as the protection scope of the present disclosure.

## Claims

1. A preparation method for the contrast agent, **characterized in that** the preparation method of the contrast agent comprises the following steps:
obtaining ex-vivo blood containing oxyhemoglobin, wherein the ex-vivo blood is autologous blood of a subject or qualified ex-vivo blood that has the same blood type of the subject and satisfies clinical standard allogeneic blood transfusion conditions;
introducing carbon monoxide into the ex-vivo blood until the oxyhemoglobin is saturated with the carbon monoxide, so as to obtain a contrast agent comprising carboxyhemoglobin.

2. The preparation method for the contrast agent according to claim 1, wherein obtaining ex-vivo blood containing oxyhemoglobin comprises the following step:
collecting ex-vivo blood that meets a preset specification by using preconfigured blood collection devices and venous blood collection method.

3. The preparation method for the contrast agent according to claim 2, wherein before introducing carbon monoxide into the ex-vivo blood until the oxyhemoglobin is saturated with the carbon monoxide, it comprises the following step:
providing light protection for the ex-vivo blood.

4. The preparation method for the contrast agent according to claim 1, wherein introducing carbon monoxide into the ex-vivo blood until the oxyhemoglobin is saturated with the carbon monoxide comprises the following steps:
generating carbon monoxide by using a carbon monoxide gas generating device;
introducing the carbon monoxide into the ex-vivo blood and allowing the ex-vivo blood not to spill until all of the oxyhemoglobin contained in the ex-vivo blood is saturated with the carbon monoxide, and then stopping the introduction of carbon monoxide.

5. The preparation method for the contrast agent according to claim 4, wherein after stopping the introduction of carbon monoxide, it comprises the following step:
discharging undissolved carbon monoxide.

6. The preparation method for the contrast agent according to claim 5, wherein discharging undissolved carbon monoxide comprises the following step:
discharging undissolved carbon monoxide by using an inert gas after stopping the introduction of carbon monoxide.

7. The preparation method for the contrast agent according to claim 6, wherein the inert gas is nitrogen or helium.

8. The preparation method for the contrast agent according to claim 1, wherein after obtaining a contrast agent comprising carboxyhemoglobin, it comprises the following step:
isolating the contrast agent from outside air, and storing it in a preset environment for later use.

9. A contrast agent, **characterized in that** the contrast agent comprises carboxyhemoglobin.

10. A contrast agent according to claim 9, **characterized in that** the contrast agent can be applied in magnetic resonance imaging examination.
